# DEMANDE DE BREVET EUROPEEN

(11) **EP 4 461 300 A1**
(43) Date de publication de la demande: **13.11.2024**
(21) Numéro de dépôt: 24168078.4
(22) Date de dépôt: 02.04.2024
(51) Int. Cl.: A61K 33/08, A61P 11/00

(54) **NO INHALÉ POUR TRAITER UN PATIENT ADULTE ATTEINT DE SDRA SÉVÈRE**

(30) Priorité: 10.05.2023 FR 2304626
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: LECOURT, Laurent, 92220 Bagneux (FR)
(74) Mandataire: Air Liquide

(57) **Abrégé**

L'invention concerne un médicament gazeux contenant du monoxyde d'azote (NO) gazeux, tel un mélange NO/azote, pour une utilisation dans le traitement d'un patient atteint d'un syndrome de détresse respiratoire aiguë (SDRA) comprenant une administration par inhalation dudit médicament gazeux audit patient. Le patient est un adulte atteint d'un SDRA sévère se caractérisant par un index de sévérité (PaO₂/FiO₂) inférieur à 80 mmHg ; une pression partielle de dioxyde de carbone (PaCO₂) d'au moins 60 mm Hg et un pH sanguin inférieur à 7.25. De préférence, le médicament gazeux contient de 200 à 2000 ppmv de NO et de l'azote pour le reste.

## Description

L'invention concerne un médicament gazeux contenant du monoxyde d'azote (NO), en particulier un mélange NO/azote, administrable par inhalation pour traiter une personne adulte, i.e. un patient adulte, atteint d'un syndrome de détresse respiratoire aiguë (SDRA) sévère, plus précisément une sous-population de patients SDRA sévère toujours hypoxémique, malgré une prise en charge ventilatoire adaptée.

Les patients atteints de syndrome de détresse respiratoire aiguë ou SDRA (ARDS en anglais) représentent environ 10% des patients hospitalisés en service de réanimation. Avant la pandémie liée au Covid-19, le nombre d'incidence du SDRA dans la population générale adulte était comprise entre 5 et 86 pour 100,000. La mortalité liée au SDRA dans cette population adulte était forte, puisque comprise entre 24% et 60% en fonction de l'âge des patients et de leur état de santé général. Le SDRA constitue donc un sérieux problème de santé publique.

Le SDRA se caractérise par une insuffisance respiratoire grave engendrée par une atteinte pulmonaire inflammatoire diffuse aiguë conduisant à une augmentation de la perméabilité des capillaires alvéolaires et à un oedème pulmonaire dit lésionnel. Autrement dit, il engendre un processus inflammatoire pulmonaire qui conduit à un oedème pulmonaire riche en protéinés non-hydrostatique, et à des conséquences sanitaires négatives pour les patients qui en sont atteints, à savoir une hypoxémie prononcée, une chute de la compliance pulmonaire et une augmentation des shunts et espaces morts intra-pulmonaires.

Le SDRA n'est, à proprement parler, pas une maladie mais un syndrome s'illustrant par de nombreux critères cliniques, paracliniques et physiologiques.

Selon une définition récente, connue en tant que « Définition de Berlin », provenant d'un groupe de travail de la société européenne de soins intensifs ou European Society of Intensive Care (ESICM), le SDRA est avéré si, chez un patient, on assiste à la présence, pendant 7 jours consécutifs, d'une atteinte clinique connue ou de symptômes respiratoires nouveaux ou qui s'aggravent, d'une combinaison d'hypoxémies aiguës se traduisant par un index de sévérité PaO₂/FiO₂ ≤ 300 mmHg, pour un patient ventilé artificiellement avec une pression expiratoire positive ou PEP (en anglais : *positive end-expiratory pressure* ou *PEEP*) d'au moins 5 cmH2O, et des opacités bilatérales qui ne sont pas totalement expliquées par une insuffisance cardiaque gauche ou une surcharge hydrique.

Toujours selon la « Définition de Berlin », on utilise l'index de sévérité PaO₂/FiO₂ pour distinguer les sévérités de SDRA, à savoir :
- SDRA léger : 200 mmHg < PaO₂/FiO₂ ≤ 300 mmHg,
- SDRA modéré : 100 mmHg < PaO₂/FiO₂ ≤200 mmHg,
- SDRA sévère : PaO₂/FiO₂ ≤ 100 mmHg.

Actuellement, pour tenter de traiter le SDRA, la stratégie thérapeutique principale vise à assurer des échanges gazeux adéquats tout en minimisant les risques de lésions pulmonaires induites par le ventilateur médical (en anglais : *Ventilator Induced Lung Injury* ou *VILI*) utilisé pour ventiler le patient.

Autrement dit, pour les traiter, les patients atteints de SDRA sont généralement ventilés mécaniquement et, si la ventilation est insuffisante ou peu efficace, le traitement peut être complété par des actions thérapeutiques additionnelles non-pharmacologiques comme une ventilation dite en décubitus ventral, le patient étant mis quelques heures par jour sur le ventre ou une circulation extracorporelle (CEC), ou des actions pharmacologiques.

Ainsi, en tant qu'action thérapeutique pharmacologique adjuvante, l'utilisation de médicaments vasodilatateurs pulmonaires avec notamment le monoxyde d'azote inhalé (NOi), tel un mélange NO/azote, a déjà été proposé pour traiter des patients en insuffisance respiratoire souffrant de SDRA, quelle qu'en soit l'origine, i.e. infections, traumatique ou autre, tels des patients infectés par un coronavirus, telle COVID-19, ou un SDRA d'une autre étiologie.

Jusqu'à présent, l'efficacité du iNO dans le traitement du SDRA se limite à une amélioration de l'oxygénation artérielle et/ou des pressions artérielles pulmonaires mais sans impact sur le devenir du patient en réanimation, en particulier en termes de mortalité/survie.

Dès lors et suite à la revue générale des études sur le iNO dans le SDRA, rapportée par F. Gebistorf et al, Inhaled nitric oxide for acute respiratory distress syndrome (ARDS) in children and adults ; Cochrane Database of Systematic Reviews 2016, Issue 6. Art. No.: CD002787), le iNO n'est en général pas recommandé, voire même déconseillé, dans les recommandations de prise en charge du SDRA.

Par conséquent, aucune autorisation de mise sur le marché (AMM) portant sur le iNO ne comprend une telle indication thérapeutique, comme en atteste le Résumé des Caractéristiques du Produit (RCP) du médicament gazeux à base de NO dénommé *INOMAX*^{®} de *INO Therapeutics*^{®}, datant de 1999, dont le paragraphe 14.2 consacré à l'usage de NO dans le SDRA s'intitule : *Ineffective in Adult Respiratory Distress Syndrome (ARDS).* En d'autres termes, là aussi, le NO inhalé n'est pas recommandé pour le traitement du SDRA chez l'adulte car considéré comme non-efficace.

Par ailleurs, on connait aussi WO2012/061264 qui propose d'utiliser du NO pour traiter un SDRA avec administration du NO par inhalation à faible dose, i.e. 0.01 à 100 ppm, pendant une période de 28 jours à 7 mois pour améliorer la fonction pulmonaire sur des patients ayant un index de sévérité PaO₂/FiO₂ pouvant atteindre 250. Toutefois, les patients testés, à savoir des enfants, présentent un index de sévérité modéré, selon la « Définition de Berlin », puisque leur index PaO₂/FiO₂ est de 140.5 +/- 43.4. Aucune amélioration de la mortalité et des effets à court terme (i.e. 6 mois) des patients n'est observée. A plus long terme, les résultats ne sont pas significatifs. D'ailleurs, le document souligne que des études « long-terme » complémentaires seront nécessaires. On comprend que ce document est limité à une population pédiatrique affectée par un SDRA plutôt modéré et montre des effets sur l'oxygénation mais pas d'effet sur une amélioration de la survie (i.e. mortalité) des patients.

De façon analogue, WO2015/106115 propose d'utiliser du NO inhalé pour traiter un SDRA chez l'enfant de moins de 16 ans avec administration du NO à très faible dose, i.e. moins de 10 ppm, pendant 2 jours à 2 mois. Là aussi, les patients testés présentent un index de sévérité modéré PaO₂/FiO₂ de 140.5 +/- 43.4. Là encore, aucune amélioration de la mortalité à court terme des patients n'est observée.

On peut citer par ailleurs FR2990858 et WO2013/175087 qui proposent d'utiliser le NO inhalé pour traiter différents pathologies pulmonaires dont le SDRA. Ces documents sont très généralistes et ne s'intéressent à aucune population de patients particulière. Ils n'apportent rien de plus que les documents précédents.

D'autres articles scientifiques existants concluent eux aussi à la non-observation d'une quelconque amélioration significative de la mortalité des patients atteints de SDRA traités par inhalation de NO (i.e. iNO). Il est même généralement préconisé de ne pas utiliser de iNO chez ces patients du fait de l'absence d'évidence de toute amélioration de leur mortalité devant le risque de troubles rénaux qui peuvent résulter de l'usage de iNO. A ce titre, on peut citer :
- Intensive Care Medecine, Springer-Verlag 2004 ®, Consenus Conférence, Inhaled Nitric Oxide therapy in neonates and children: reaching a European consensus, D.J. Macrae et al, published 13 Jan. 2004*, parag. : Used of inhaled nitric oxide in paediatric acute lung injury and acute respiratory distress syndrom,* qui souligne à une non-efficacité du iNO sur la mortalité et une amélioration uniquement transitoire sur l'oxygénation;
- Pilbeam, Mechanical ventilation, 4th Edition, Spécial Technics in Mechanical Ventilation, Section IV : Nitric Oxide, 2006® Mosby Inc., *parag. ARDS,* qui rapporte que les résultats de 4 grandes études n'ont montré aucun bénéfice du iNO sur la mortalité des patients malgré une amélioration de l'oxygénation.

En fait, il ressort de la plupart de ces publications que les patients testés souffraient de SDRA de sévérité variable selon la classification de Berlin, en général modérée ou légère, et qu'ils n'avaient pas reçu avant la mise sous iNO, de ventilation assistée adaptée, typiquement de ventilation protectrice avec mise du patient sur le ventre pour limiter la pression exercée sur ses poumons et améliorer les échanges gazeux.

D'une façon générale, l'ensemble des études réalisées jusqu'à présent pour évaluer les effets du NO inhalé dans le traitement du SDRA a montré, au mieux, une amélioration transitoire de l'oxygénation artérielle mais celle-ci n'est jamais corrélée à une amélioration de la survie (i.e. taux de mortalité) des patients testés.

On connait par ailleurs la publication : Syndrome de Détresse Respiratoire Aigüe, de J-C. Chevrolet et al., EMC-Pneumologie 1 (2004), 143-186 ; qui adresse un état des lieux des connaissances sur le SDRA, parle notamment de l'index de sévérité et liste différentes méthodes de traitement pouvant être envisagées comme traitement. Le NO inhalé en fait partie. Toutefois, ce document classe le NO comme traitement de routine non-recommandé pour le SDRA (voir Tableau 4 en p. 170), indique qu'il convient de rester prudent par rapport à une telle utilisation du NO tout en précisant qu'aucune étude n'avait démontré un quelconque effet du NO sur la survie des patients (p.181). Il conclut que malgré tous les progrès réalisés dans le traitement du SDRA, aucune thérapeutique actuelle n'est utilisable au lit du malade pour traiter le SDRA. Dès lors, ce document ne préconise pas l'usage du NO mais, au contraire, dissuade de l'utiliser étant donné qu'il est présenté comme ne présentant aucun effet améliorant la survie des patients.

Un problème est donc de pouvoir améliorer l'oxygénation artérielle d'un (ou de plusieurs) patient mais aussi son taux de survie, c'est-à-dire diminuer sa mortalité, en particulier pour un patient adulte atteint d'un SDRA sévère présentant des critères de sévérité, à savoir ceux faisant partie des critères de mises sous ECMO selon l'étude dite « EOLIA », référencée : A. Combes et al ; Extracorporeal Membrane Oxygénation for Severe Acute Respiratory Distress Syndrome ; New Engl. J. Med. ; 2018; 378:1965-75*.*

Une solution selon l'invention porte sur un médicament gazeux contenant du monoxyde d'azote (NO) gazeux pour une utilisation dans le traitement d'un patient adulte atteint d'un syndrome de détresse respiratoire aiguë (SDRA) comprenant une administration par inhalation dudit médicament gazeux audit patient.

Selon l'invention, le patient est un adulte atteint d'un SDRA sévère se caractérisant par :
i) un index de sévérité tel que : PaO₂/FiO₂ ≤ 80 mmHg et/ou
ii) une pression partielle de dioxyde de carbone (PaCO₂) et un pH sanguin tels que : PaCO₂ ≥ 60 mm Hg et pH < 7.25,
où : PaO₂, PaCO₂ et le pH sont mesurés chez le patient avant administration du médicament gazeux.

Dans le cadre de l'invention, on considère que l'état de santé du patient s'est amélioré grâce à l'administration de iNO, si on constate qu'il ne présente plus les critères de mise sous ECMO, à savoir :
- une amélioration de son index de sévérité, c'est-à-dire PaO₂/FiO₂ > 80 mmHg ou
- une PaCO₂ < 60 mmHg et un pH ≥ 7.25.

Pour déterminer l'index de sévérité d'un patient, les valeurs de PaO₂, de PaCO₂ et de pH sont mesurées, déterminées ou évaluées chez le patient considéré, c'est-à-dire le patient à traiter, avant toute administration du médicament gazeux, i.e. du médicament gazeux contenant du NO gazeux selon l'invention.

Comme indiqué ci-après, la PaCO₂ et la PaO₂ sont déterminées par gazométrie artérielle ou « gaz du sang », typiquement via une prise de sang réalisée avant toute administration du médicament gazeux à base de NO, c'est-à-dire avant traitement par NOi. De tels paramètres sont classiquement relevés, notamment après mise du patient sous oxygène (i.e. administration d'une FiO₂ donnée), par exemple lors de sa prise en charge.

D'une façon générale, les gaz du sang permettant de suivre les échanges gazeux du patient en fournissant les valeurs de PaO₂ de PaCO₂ et les saturations. Les valeurs peuvent être relevées plusieurs fois par jour dans un service de réanimation, notamment en fonction de la gravité de l'état du patient. Le relevé des gaz du sang mentionne aussi la FiO₂ puisque les valeurs de PaO₂ et de saturation notamment sont corrélées à la proportion d'oxygène apportée au patient, i.e. la FiO₂.

La fraction inspirée en oxygène ou FiO₂ est la quantité d'oxygène (vol.%) présente dans le mélange gazeux inhalé par la patient, c'est-à-dire la quantité d'oxygène fournie au patient. Elle correspond à la concentration en O₂ fixée par le médecin et apportée par le ventilateur médical. La FiO₂ peut atteindre 100% O₂ pour un patient très affecté, c'est-à-dire correspondre à un flux d'oxygène pur. Typiquement, la FiO₂ chez les patients SDRA est comprise en fonction de la sévérité du SDRA entre 50 et 100 vol.% d'oxygène.

Dit autrement, dans le cadre de l'invention, seuls les patients présentant un SDRA sévère présentant les paramètres gazométriques suivants : un index de sévérité (raport PaO₂/FiO₂ ) inférieur ou égal à 80 mmHg ou une pression partielle de dioxyde de carbone (PaCO₂) telle que : PaCO₂ ≥ 60 mmHg et un pH sanguin tel que : pH < 7.25 où la PaCO₂ et le pH sont mesurés chez le patient avant administration du médicament gazeux contenant du NO gazeux selon l'invention, sont concernés étant donné que ces paramètres gazométriques correspondent à des critères de mise sous ECMO (étude EOLIA) qui est généralement le dernier recours après échec d'une ventilation conventionnelle (ECMO pour *extracorporeal membrane oxygénation* ou oxygénation membranaire extracorporelle).

L'ECMO est une technique d'oxygénation directe du sang par mise en circulation extracorporelle (CEC) du patient avec utilisation d'une pompe pour générer un débit sanguin extracorporel et d'une (des) membrane d'oxygénation pour schématiquement débarrasser le sang du CO₂ qui s'y trouve et enrichir le sang en oxygène.

En effet, il a été mis en évidence dans le cadre de la présente invention que, contrairement à ce qui est habituellement admis dans l'art antérieur, à savoir que le NO n'a pas d'effet significatif sur la survie/mortalité des patients atteints de SDRA, de façon surprenante, une administration de iNO améliore significativement la survie d'une sous-population particulière de patients adultes, à savoir les patients les plus gravement atteints de SDRA, c'est-à-dire de SDRA sévère, de différentes étiologies, par exemple les patients atteints d'une pneumonie d'origine virale ou bactérienne, notamment ceux infectés par un virus, tel un coronavirus, par exemple le COVID-19, ou autres.

En effet, jusqu'à la présente invention, aucune étude n'avait démontré, une efficacité du NO inhalé sur le taux de survie de patients adultes atteints d'un SDRA sévère, quelles que soient les populations de patients SDRA étudiées.

Dans le cadre de l'invention :
- PaO₂ : est la pression partielle d'oxygène (O₂) reflète la quantité d'oxygène dans le sang d'une personne. Elle est déterminée par gazométrie artérielle (i.e. prise de sang). Elle est exprimée ici en mmHg.
- FiO₂ : est la fraction inspirée d'oxygène est la quantité ou proportion d'oxygène présent dans le gaz administré par inhalation à une personne et est exprimée en vol.%. Elle est fixée au niveau du ventilateur médical par le personnel soignant. Pour les patients SDRA, elle est généralement d'au moins 40%vol., voire même d'au moins 50%vol. et peut atteindre environ 100%vol., ce qui correspond à de l'oxygène (quasi)pur.
- PaCO₂ : est la pression partielle de dioxyde de carbone (CO₂) reflète la quantité ou proportion de CO₂ dans le sang artériel d'une personne. Elle est déterminée par gazométrie artérielle. Elle est exprimée ici en mmHg.
- l'index de sévérité PaO₂/FiO₂ reflète le degré d'oxygénation d'un patient sous ventilation artificielle ou assistée, c'est-à-dire le rapport d'oxygénation artérielle (i.e. quantité d'oxygène dans le sang) en fonction de la quantité d'oxygène apportée par la ventilation assistée, i.e. par le ventilateur médical. A titre indicatif, une valeur « normale » est de l'ordre de 450 mmHg. Il est exprimé ici en mmHg. Ce rapport, comme vu précédemment, est un indicateur permettant de définir les stades de gravité du SDRA selon la définition de Berlin.
- le pH reflète le degré d'acidité ou d'alcalinité du sang. A titre indicatif, un pH normal se situe entre 7,35 et 7,45.
- ppmv : signifie partie par million en volume.
- %vol. : signifie pourcentage en volume.
- air : on considère qu'il contient environ 20 à 21%vol. d'oxygène (O₂) et environ 78% d'azote (N₂). Les autres espèces présentes (i.e. argon, CO₂, hélium...) sont considérées comme des impuretés inévitables n'ayant pas d'influence sur l'efficacité du traitement par iNO ; elles sont donc ignorées.

Selon le mode de réalisation considéré, le médicament gazeux utilisé selon l'invention peut comprendre l'une ou plusieurs des caractéristiques suivantes :
- il est formé d'un mélange de NO et d'azote (N₂).
- il est formé d'un mélange de NO et d'azote contenant de 200 à 2000 ppmv de NO, de préférence comprise entre 200 et 1500 ppmv.
- il est formé d'un mélange de NO et d'azote contenant de 200 à 1000 ppmv, de préférence comprise entre 400 et 900 ppmv de NO, le reste étant de l'azote.
- avantageusement, il est formé d'un mélange de NO et d'azote contenant 800 ppmv de NO, le reste étant de l'azote.
- alternativement, il est formé d'un mélange de NO et d'azote contenant 450 ppmv de NO, le reste étant de l'azote.
- alternativement encore, il est formé d'un mélange de NO et d'azote contenant 225 ppmv de NO, le reste étant de l'azote.
- il est dilué (avant inhalation par le patient) avec un gaz respiratoire contenant de l'oxygène, c'est-à-dire avant administration au patient.
- il est dilué (avant inhalation par le patient) avec un gaz respiratoire contenant au moins 20%vol. environ d'oxygène, de préférence au moins 21%vol. environ d'oxygène, c'est-à-dire avant administration au patient, de préférence au moins 50% d'O₂.
- il est dilué (avant inhalation par le patient) avec un gaz respiratoire contenant de l'oxygène, avant administration au patient, par exemple un flux d'oxygène pur ou un mélange O₂/N₂.
- il est dilué avec un gaz respiratoire pour obtenir un mélange final (i.e. gaz inhalé par le patient) contenant de l'oxygène, de l'azote et moins de 40 ppmv de NO en tant que dose de NO à administrer, c'est-à-dire en tant que posologie.
- de préférence la dose de NO à administrer (dans le mélange final après dilution) est comprise entre 1 et 30 ppmv, de préférence encore de de 1 à 20 ppmv de NO, avantageusement de l'ordre de 10 ppmv.
- le gaz respiratoire contenant de l'oxygène est fourni par un ventilateur médical.
- le gaz respiratoire contenant de l'oxygène est un mélange O₂/N₂ ou de l'oxygène pur.
- le patient est un adulte âgé d'au moins 18 ans, de préférence âgé d'au moins 30 ans, de préférence âgé d'au moins 40 ans, typiquement âgé de 45 à 95 ans, de préférence âgé entre 50 et 90 ans.
- il est administré par inhalation pendant plusieurs heures, de préférence au moins 24h, typiquement entre 24h et 96h, voire plus dans les cas les plus graves.
- il est administré par inhalation au moyen d'une sonde d'intubation trachéale (i.e. le patient est intubé).
- il est conditionné dans (au moins) un récipient de gaz, typiquement une bouteille de gaz.
- alternativement, il est produit sur site (i.e. de manière extemporanée) au moyen d'un dispositif générateur de NO, comme par exemple celui décrit par EP3509684.
- le (les) récipient de gaz, typiquement une bouteille de gaz, contient un mélange NO/N₂ constitué de 450 ppmv ou 800 ppmv de NO et d'azote pour le reste (et éventuellement des impuretés inévitables).
- le patient est sous ventilation artificielle et intubé, c'est-à-dire sous ventilation invasive, typiquement en réanimation.
- le patient à traiter a besoin d'un traitement par inhalation de NO.
- le patient est alité, i.e. couché, pendant l'inhalation de iNO.
- le patient est un homme ou un femme, ou autre.
- le patient présente (avant traitement) une hypoxémie réfractaire malgré une ventilation assistée et optionnellement une mise en position ventrale (i.e. décubitus ventral), c'est-à-dire que hypoxémie réfractaire n'est pas traitable par ventilation assistée et décubitus ventral.
- le patient présente une ou des comorbidités choisies parmi une hypertension systémique, un diabète, une immunodéficience et une bronchopneumopathie chronique obstructive (BPCO).
- le NO est le principe actif du médicament gazeux.
- le (les) récipient de gaz, typiquement une bouteille de gaz, contient le mélange NO/N₂ comprimé à une pression d'au moins 150 bar abs, typiquement de l'ordre de 200 bar abs, mesurée lorsque le récipient est plein, c'est-à-dire avant toute utilisation du gaz.
- le patient est atteint d'un SDRA sévère se caractérisant par un index de sévérité tel que : 35 mmHg ≤ PaO₂/FiO₂ ≤ 80 mmHg.
- le patient est atteint d'un SDRA sévère se caractérisant par un index de sévérité tel que : 40 mmHg ≤ PaO₂/FiO₂ ≤ 80 mmHg.
- le patient est atteint d'un SDRA sévère se caractérisant par un index de sévérité tel que : 50 mmHg ≤ PaO₂/FiO₂ ≤ 80 mmHg.
- le patient est atteint d'un SDRA sévère se caractérisant par un index de sévérité tel que : 60 mmHg ≤ PaO₂/FiO₂ ≤ 80 mmHg.

L'invention concerne aussi une installation de ventilation pour fournir une assistance ventilatoire à un patient adulte atteint d'un SDRA sévère, comprenant :
- au moins une bouteille de gaz, i.e. récipient de gaz comprimé, contenant un médicament gazeux selon l'invention formé d'un mélange NO/N₂,
- un ventilateur médical pour fournir un flux de gaz respiratoire contenant de l'oxygène (i.e. au moins 21%vol. environ), à un circuit patient, et
- un appareil de fourniture de gaz alimenté en mélange NO/N₂ par ladite au moins une bouteille de gaz, et relié fluidiquement audit circuit patient pour injecter le mélange NO/N₂ dans le flux de gaz respiratoire contenant de l'oxygène circulant dans le circuit patient, c'est-à-dire pour opérer un mélange entre le flux de NO/N₂ et le flux de gaz respiratoire contenant de l'oxygène au sein dudit circuit patient.

Selon le mode de réalisation considéré, l'installation de ventilation de l'invention peut comprendre l'une ou plusieurs des caractéristiques suivantes :
- la bouteille de gaz contient un mélange NO/N₂ contenant de 100 à 2000 ppmv de NO et de l'azote pour le reste, de préférence de 100 à 1500 ppmv, de préférence de 100 à 1000 ppmv, typiquement 450 ou 800 ppmv de NO.
- le flux de gaz respiratoire contenant de l'oxygène est un mélange O₂/N₂ (e.g. au moins 40%vol., voire au moins 50%vol.) ou de l'oxygène pur.
- le circuit patient véhicule un flux gazeux final contenant une proportion ou dose de NO souhaitée comprise entre 1 et 40 ppmv, de préférence moins de 20 ppmv de NO environ.
- le circuit patient véhicule un flux gazeux final contenant une proportion ou dose de NO souhaitée de l'ordre de 10 ppmv.
- le flux gazeux final contenant la dose de NO souhaitée est obtenu par injection du mélange NO/N₂ provenant de l'appareil de fourniture de gaz dans le flux de gaz respiratoire contenant de l'oxygène provenant du ventilateur médical.
- le circuit patient alimente une sonde d'intubation trachéale avec le flux gazeux final contenant la dose de NO souhaitée.
- le circuit patient comprend une branche inspiratoire et une branche expiratoire.
- la branche inspiratoire et la branche expiratoire sont raccordées fluidiquement à une pièce de raccordement ou de jonction, telle une pièce en Y.
- la sonde d'intubation trachéale est reliée fluidiquement à la pièce de raccordement, i.e. la pièce en Y.
- la branche inspiratoire est raccordée fluidiquement, en amont, au ventilateur médical et, en aval, à la sonde d'intubation trachéale, via la pièce de raccordement, i.e. la pièce en Y, de manière à acheminer notamment le flux gazeux final contenant le NO à la dose souhaitée.
- la branche expiratoire est raccordée fluidiquement, en amont, à la sonde d'intubation trachéale, via la pièce de raccordement, i.e. la pièce en Y, et, en aval, au ventilateur médical de manière à acheminer le gaz sortant des poumons du patient (i.e. gaz riche en CO₂) jusqu'au ventilateur médical.
- la branche inspiratoire comprend un humidificateur de gaz permettant d'ajouter de la vapeur d'eau au flux gazeux final contenant le NO à la dose souhaitée.
- la branche inspiratoire comprend en outre un capteur de débit relié électrique à l'appareil de fourniture de gaz de manière à lui fournir des mesures de débit du flux de gaz respiratoire contenant de l'oxygène provenant du ventilateur médical.
- l'appareil de fourniture de gaz comprend une interface graphique utilisateur (IGU) comprenant un afficheur graphique.
- l'afficheur graphique est configuré pour afficher une ou des touches de sélection virtuelle ou analogue actionnable par l'utilisateur, en particulier par appui digital.
- l'afficheur graphique comprend une dalle tactile.
- l'afficheur graphique est à affichage en couleurs.
- l'IGU est configurée pour permettre à l'utilisateur de fixer ou sélectionner une dose de NO souhaitée, en particulier via une ou des touches de sélection virtuelle affcihée(s) sur l'afficheur graphique.
- l'appareil de fourniture de gaz comprend des moyens de pilotage configurés pour traiter les mesures de débit provenant du capteur de débit et piloter la délivrance du flux de mélange NO/N₂ en réponse au traitement des mesures de débit opéré et en fonction de la dose de NO souhaitée fixée ou sélectionnée par l'utilisateur.
- l'appareil de fourniture de gaz et le ventilateur médical sont alimentés en courant électrique, notamment par du courant secteur (110/220V) et/ou une (des) batterie rechargeable interne.

Une telle installation de ventilation est utilisée pour traiter un patient adulte atteint d'un SDRA sévère comme décrit ci-dessus, ledit traitement comprenant une administration par inhalation audit patient, d'une dose souhaitée de NO inférieure à 40 ppmv, typiquement inférieure à 20 ppmv, via une sonde d'intubation trachéale.

L'invention concerne en outre une méthode de traitement d'un patient adulte, i.e. une personne adulte, atteint d'un syndrome de détresse respiratoire aiguë (SDRA) sévère se caractérisant par un index de sévérité tel que : PaO₂/FiO₂ ≤ 80 mmHg, ou une pression partielle de dioxyde de carbone (PaCO₂) et un pH sanguin tels que : PaCO₂ ≥ 60 mm Hg et pH < 7.25, où : PaO₂, PaCO₂ et le pH sont mesurés chez le patient avant administration du médicament gazeux, dans laquelle on administre par inhalation au patient à traiter un médicament gazeux contenant du monoxyde d'azote (NO) gazeux selon l'invention, c'est-à-dire un mélange gazeux NO/N₂, de manière à améliorer l'oxygénation sanguine du patient et augmenter ses chances de survie (i.e. diminuer son risque de décès).

Selon le mode de réalisation considéré, la méthode de traitement de l'invention peut comprendre l'une ou plusieurs des caractéristiques suivantes :
- on identifie un patient atteint d'un syndrome de détresse respiratoire aiguë (SDRA) sévère se caractérisant par un index de sévérité ou une pression partielle de dioxyde de carbone (PaCO₂) et un pH sanguin comme définit ci-dessus.
- l'identification du patient atteint de SDRA comprend la réalisation d'une gazométrie artérielle ou « gaz du sang » chez le patient, notamment pour déterminer la PaCO₂ et la PaO₂.
- le patient présente (avant traitement) une hypoxémie réfractaire malgré une ventilation assistée et/ou une mise en position couchée ventrale (i.e. décubitus ventral).
- le patient est sous ventilation artificielle invasive et intubé, typiquement il est hospitalisé dans un service de réanimation d'un hôpital ou analogue.
- le patient présente une ou des comorbidités choisies parmi une hypertension, un diabète, une immunodéficience et une bronchopneumopathie chronique obstructive (BPCO).
- le patient à traiter a besoin d'un traitement par inhalation de NO.
- le patient est alité, i.e. couché, pendant l'inhalation de iNO.
- le patient est un homme ou une femme, ou autre.
- le patient est un adulte âgé d'au moins 18 ans, de préférence âgé d'au moins 30 ans, de préférence âgé d'au moins 40 ans, typiquement âgé de 45 à 90 ans, de préférence âgé entre 50 et 80 ans.
- le médicament gazeux est administré par inhalation au moyen d'une sonde d'intubation trachéale (i.e. le patient est intubé).
- le médicament gazeux est administré une mise en place de la sonde d'intubation trachéale (i.e. de préférence, rapidement après intubation du patient).
- le médicament gazeux est formé d'un mélange de NO et d'azote (N₂), c'est-à-dire un mélange gazeux NO/N₂.
- le mélange constitué de NO et d'azote contient de 200 à 2000 ppmv de NO, de préférence de 200 à 1000 ppmv de NO, de préférence encore entre 400 et 900 ppmv de NO, le reste étant de l'azote.
- avantageusement, le mélange de NO et d'azote contient 800 ppmv de NO, le reste étant de l'azote.
- alternativement, le mélange de NO et d'azote contient 225 ppmv de NO, le reste étant de l'azote.
- alternativement encore, le mélange de NO et d'azote contient 450 ppmv de NO, le reste étant de l'azote.
- le médicament gazeux NO/N₂ est dilué avec un gaz respiratoire contenant (au moins 20%vol.) de l'oxygène, avant administration au patient, de manière à obtenir un mélange final contenant de l'oxygène, de l'azote et moins de 40 ppmv de NO en tant que dose de NO à administrer, c'est-à-dire en tant que posologie, de préférence environ au moins 21%vol. d'oxygène.
- de préférence la dose de NO à administrer au patient est comprise entre 1 et 30 ppmv, de préférence encore de 1 à 20 ppmv de NO, avantageusement de l'ordre de 10 ppmv.
- le gaz respiratoire contenant de l'oxygène est fourni par un ventilateur médical, de préférence il est un mélange O₂/N₂ ou de l'O₂ pur.
- le médicament gazeux est administré par inhalation pendant plusieurs heures, de préférence au moins 24h, typiquement entre 24h et 96h, voire plus dans les cas les plus graves.
- l'administration de iNO est stoppée progressivement (en fin de traitement) en diminuant la dose de NO (par exemple 10 ppmv) de 1 ppmv toutes les 30 min à 60 min.
- l'administration de iNO est stoppée progressivement en surveillant l'oxygénation du patient, c'est-à-dire selon un protocole de sevrage comprenant une diminution progressive de la dose de NO administrée pour éviter les risques d'effet rebond liés à un arrêt brutal d'une administration de NO inhalé.
- l'administration de iNO est stoppée après une durée de traitement par iNO donnée, par exemple une durée maximale de l'ordre de plusieurs jours ou moins.
- alternativement, l'administration de iNO est stoppée lorsque l'état de santé du patient s'est amélioré, en particulier après amélioration des échanges gazeux et retour à une oxygénation artérielle correcte

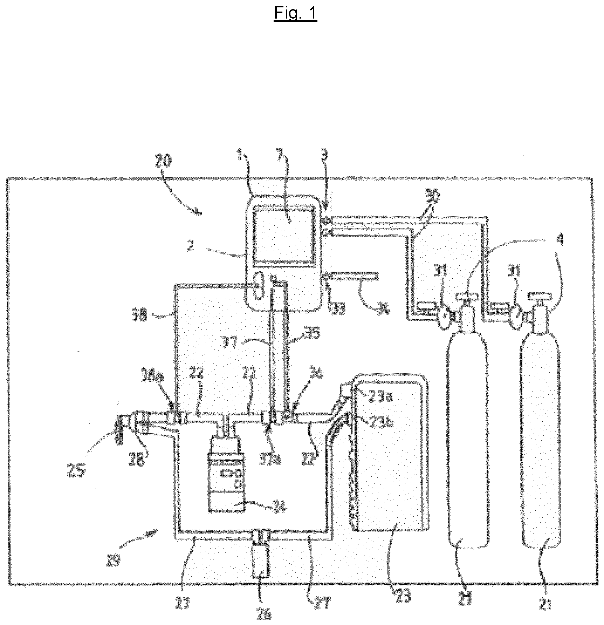

L'invention va maintenant être mieux comprise grâce à la description détaillée suivante, faite à titre illustratif mais non limitatif, rapportant les résultats obtenus au cours d'essais comparatifs réalisés chez des patients atteints de SDRA et en référence à la figure annexée où la Figure 1 représente un mode de réalisation d'une installation de ventilation permettant de fournir du iNO à un patient.

Afin de montrer l'efficacité du médicament gazeux inhalable à base de NO selon l'invention, une étude a été réalisée. Dans le cadre de cette étude, on a sélectionné 300 personnes, i.e. hommes et femmes, appelées « patients », qui étaient atteints de SDRA et traités par du NOi dans plusieurs hopitaux. Ces patients SDRA ont été classés en fonction de leur sévérité grâce à une évaluation de leurs paramètres d'oxygénation, à savoir PaCO₂, de PaO₂ et pH sanguin, avant toute administration de iNO. Sur cette population, une sous population de patients les plus graves a été définie incluant ceux ayant des critères de recours à une ECMO.

Ainsi, parmi ces 300 patients, seule une sous-population de 62 patients présente ces critères de mise sous ECMO, appelés « patients dit ECMO », à savoir un rapport PaO₂/FiO₂ ou index de sévérité) tel que : PaO₂/FiO₂ ≤ 80 mmHg ou ayant une pression partielle de dioxyde de carbone PaCO₂ telle que : PaCO₂ ≥ 60 mm Hg et un pH sanguin tel que pH < 7.25. Cette sous-population de 62 « patients ECMO » comprenait 49 hommes et 13 femmes âgés de 53 à 71 ans, avec un âge moyen de 66 ans.

Ces patients sont intubés et ventilés par un ventilateur médical. Pour ce faire, on a utilisé une installation de ventilation telle celle décrite en Fig. 1 et détaillée ci-après. Il est à préciser que ces patients ne sont pas placés sous ECMO (*extracorporeal membrane oxygénation*), c'est-à-dire sous circulation sanguine extracorporelle, bien qu'ils présentent les critères de mise sous ECMO, mais sont néanmoins maintenus sous ventilation artificielle.

Du iNO leur a été administré par inhalation, via une sonde d'intubation trachéale, sous forme d'un mélange NO et d'azote (le reste) qui est mélangé à un gaz non-hypoxique de l'oxygène en quantité adéquate, à savoir contenant ici au moins 60% d'oxygène (reste azote), typiquement de l'oxygène pur, ce qui correspond à une FiO₂ égale à 100%.

Après dilution des gaz (i.e. NO/N₂ et ici O₂), le mélange final NO/O₂/N₂ obtenu est administré par inhalation aux patients testés ayant été intubés. Ce mélange final contient environ 10 ppmv de NO (valeur moyenne désirée), et de l'azote et de l'oxygène pour le reste. Il est toutefois à noter que des fluctuations ponctuelles ont pu être observées, lors de l'administration de iNO, conduisant à des valeurs de teneur en NO administrées comprises entre environ 6 et 13 ppmv. L'inhalation du NO gazeux se fait de façon continue pendant une durée déterminée de plusieurs heures.

Ainsi, les résultats obtenus dans les 6 heures suivant l'inhalation de iNO sont donnés dans le Tableau 1. Bien que la réponse obtenue pour chaque patient ait été évaluée à différents moments, après le début d'administration de iNO, le plus intéressant d'un point de vue clinique est d'opérer une évaluation de la réponse à au moins 6 heures, c'est-à-dire au moins 6h après début d'inhalation de iNO, car cette réponse précoce permet de visualiser un effet propre du NO administré.

La réponse au NO est considérée comme une perte des critères de mise sous ECMO après administration du iNO, dans les 6 heures suivant le début de l'inhalation du gaz à base de NO.

Le Tableau 1 compare donc les patients dits « répondeurs » (i.e. perte des critères d'ECMO dans les 6 h suivant la mise sous iNO) aux patients dits « non-répondeurs » (i.e. persistance des critères d'ECMO après administration de iNO).

**Tableau 1**

| Patients testés | Patients « non-répondeurs » présentant encore les critères ECMO | Patients « répondeurs » ne présentant plus les critères ECMO |
|---|---|---|
| 62 patients | 32 | 30 |
| % | 51.6% | 48.4% |
| Hommes | 24 | 25 |
| Femmes | 8 | 5 |
| Age moyen (ans) | 65 | 66 |
| Poids moyen (kg) | 89 | 86 |
| IMC moyen (kg/m²) | 29 | 29 |

| | | |
|---|---|---|
| IMC : indice de masse corporel | | |

Comme on le voit, parmi les 62 patients testés, 30 patients ont « répondu » positivement à l'administration de iNO, c'est-à-dire que leur état de santé s'est amélioré, soit 48.4% des patients testés.

Les patients ont été considérés comme « répondeurs » au monoxyde d'azote inhalé (iNO) lorsqu'on observe chez eux, comme critères de « réponse positive », une disparition des critères d'ECMO chez les patients, dans les 6 heures suivant une inhalation du iNO, donc une amélioration de leur oxygénation.

A l'inverse, les patients « non-répondeurs » sont ceux pour lesquels une telle amélioration n'est pas observée et qui conservent des critères de mise sous ECMO.

Ces premiers résultats montrent que le iNO peut agir efficacement sur une majorité de patients souffrant d'ARDS et présentant au départ, i.e. lors du diagnostic de leur état, les critères de mise sous ECMO. Autrement dit, ces résultats confirment l'intérêt du NO sur l'amélioration des paramètres d'oxygénation et dans le cas présent, sur une population de patients les plus graves.

Il est à noter que les caractéristiques démographiques de cette sous-population sont globalement similaires à la population globale, par exemple certains présentent des comorbidités, par exemple du type hypertension, diabète, immunodéficience ou BPCO.

Le Tableau 2 donne la répartition de ces 62 patients testés pour ce qui est de leur mise en position ventrale éventuelle entre le moment de leur intubation et le début de l'administration de iNO.

**Tableau 2**

| Mise en position ventrale | Patients « non-répondeurs » présentant encore les critères ECMO | Patients « répondeurs » ne présentant plus les critères ECMO |
|---|---|---|
| oui | 20 (62.5%) | 17 (56.7%) |
| Non | 12 (37.5%) | 13 (43.3%) |

On constate que la mise en position ventrale ne semble pas un critère déterminant chez les patients répondeurs, alors qu'elle est plus significative chez les patients non-répondeurs. Mais, là encore, il est à rappeler que les caractéristiques démographiques de cette sous-population sont globalement similaires à la population globale.

Par ailleurs, Le Tableau 3 donne l'évolution des valeurs moyennes du rapport PaO₂/FiO₂ et de la pression partielle d'oxygène PaO₂ chez les 62 patients testés, mesurées avant et après administration de iNO (i.e. après 6h).

**Tableau 3**

| En mmHg | Patients « non-répondeurs » présentant encore les critères ECMO N=32 | Patients « répondeurs » ne présentant plus les critères ECMO N=30 |
|---|---|---|
| Valeur moyenne du rapport PaO₂/FiO₂ avant administration de iNO | 68.3 (17.0) | 71.4 (27.2) |
| Valeur moyenne du rapport PaO₂/FiO₂ après administration de iNO | 68.1 (23.6) | 121.0 (38.5) |
| Evolution du rapport PaO₂/FiO₂ avant et après NO | -0,3% | 69.4% |
| Valeur de PaO₂ avant administration de iNO | 65 | 68 |
| Valeur de PaO₂ après administration de iNO | 64 | 104 |
| Evolution du la PaO₂ avant et après NO | -1,5% | 52.9% |
| Valeur moyenne de la PACO2 avant administration de iNO | 56.7 (12.9) | 49.4 (12.0) |
| Valeur moyenne de la Paco2 après administration de iNO | 57.3 (11.0) | 47.6 (8.7) |

On constate que l'administration de NO inhalé engendre une amélioration notable (env. +53% pour rapport PaO₂/FiO₂ et PaO₂) de la quantité d'oxygène dans le sang des patients « répondeurs », c'est-à-dire de leur oxygénation sanguine, alors qu'elle est sans influence chez les patients « non-répondeurs ». Ceci démontre que le iNO favorise notablement le passage de l'oxygène des poumons vers le sang des patients « répondeurs » présentant un SDRA sévère.

En outre, le Tableau 4 consigne les résultats relatifs à la mortalité des patients testés évaluée, après 6h d'administration de iNO, Cette mortalité étant jugée a 28 jours et sur la durée totale de l'hospitalisation .

**Tableau 4**

| Décès Nombre (%) | Patients « non-répondeurs » présentant encore les critères ECMO N=32 | Patients « répondeurs » ne présentant plus les critères ECMO N=30 |
|---|---|---|
| Taux de mortalité hospitalière | 84.4% | 60% |
| Taux de mortalité à 28 jours | 68.8% | 50.7% |

Les essais réalisés montrent que l'effet particulièrement notable d'une administration de iNO sur le taux de survie des 30 patients « répondeurs » présentant un SDRA sévère (après 6h d'inhalation de NO) puisque 40% de ces patients ont survécu, alors que dans le groupe des 32 patients « non-répondeurs », seuls 15.6% des patients ont survécu au final.

Autrement dit, la mortalité a diminué de maniéré significative dans le groupe des patients SDRA très sévères ayant des critères de mise sous ECMO, lesquels répondent positivement, après iNO, c'est-à-dire qu'ils ne présentent plus les critères d'ECMO dans les 6 heures suivant l'administration de iNO.

Plus généralement, parmi les 300 patients testés au départ, la mortalité « intra-hospitalière » a été de 65% dans la population totale et de 72% dans la sous-population des patients SDRA sévères (selon la Définition de Berlin).

Toutefois, parmi les patients « répondeurs » lesquels présentaient au départ les critères d'ECMO (i.e. 62 patients « ECMO »), la mortalité n'a été que de 60%, c'est-à-dire significativement inférieure à celle des patients « non-répondeurs », pour lesquels la mortalité a été de 84.4% (p=0.032), et aussi inférieure à celle de la population globale testées (300 patients) qui est de 65%.

Cette comparaison avec population globale confirme l'effet du iNO qui agit efficacement sur l'oxygénation mais aussi, de manière surprenante, sur la mortalité de la sous-population considérée.

Par ailleurs, en considérant la population globale de patients testés, on ne constate pas de modification de la survie sur cette population générale mais uniquement de l'oxygénation des patients, ce qui confirme les connaissances antérieures sur le NO dans le traitement du SDRA qui concluent à l'inefficacité du iNO dans le SDRA, en particulier sur toute amélioration de la mortalité des patients.

Les essais opérés dans le cadre de la présente invention démontrent donc l'efficacité du iNO dans le traitement d'une sous-population particulière de patients atteints de SDRA sévère se caractérisant par un index de sévérité tel que : PaO₂/FiO₂ ≤ 80 mmHg ou une pression partielle de dioxyde de carbone (PaCO₂) et un pH sanguin tels que : PaCO₂ ≥ 60 mm Hg et pH < 7.25, où : PaO₂, PaCO₂ et/ou pH sont mesurés chez le patient avant administration du médicament gazeux et FiO₂ est la fraction inspirée d'oxygène, i.e. la quantité ou proportion d'oxygène présent dans le gaz administré par inhalation au patient.

Avantageusement, l'administration du iNO est aussitôt que possible après intubation du patient, c'est-à-dire que le NO donné plus rapidement après une intubation semble être plus efficace dans cette sous-population de patients. Une administration de NO rapidement et, de préférence, dans les 24h après intubation est donc recommandée.

D'une manière générale, les résultats obtenus sont particulièrement surprenants étant donné que le iNO est réputé, selon l'art antérieur, ne pas être efficace en termes d'amélioration de la survie/mortalité des patients atteints de SDRA.

Or, les essais réalisés montrent que le iNO améliore non seulement l'oxygénation mais aussi la survie (i.e. baisse de mortalité) d'une sous-population particulière de patients souffrant de SDRA, à savoir ceux patients atteints de SDRA sévère et présentant, au départ (avant administration de iNO), des critères de mise sous ECMO comme expliqué ci-dessus. Une administration de iNO à ces patients permet d'améliorer non seulement leur état de santé, i.e. oxygénation, mais aussi leur survie, ce qui n'a jamais été démontré jusqu'ici.

D'une façon générale, l'administration de iNO à un patient atteint de SDRA sévère et présentant, au départ, des critères de mise sous ECMO, peut se faire, dans le cadre de son traitement, en utilisant une installation de ventilation 20, c'est-à-dire une installation de fourniture de gaz, comme celle schématisée en Fig. 1.

Dans le mode de réalisation présenté, l'installation de ventilation 20 comprend un appareil de délivrance de NO 1 coopérant avec un ventilateur médical 23 et à un circuit patient 29, de manière à injecter du NO dans le circuit patient 29 qui comprend une branche inspiratoire 22 et une branche expiratoire 27. La branche inspiratoire 22 véhicule en outre un gaz respiratoire contenant une forte proportion d'oxygène, typiquement de l'oxygène pur ou un mélange O₂/N₂ (par exemple contenant au moins 60%vol d'O₂) fourni par un ventilateur médical 23.

Deux bouteilles de gaz sous pression 21 contenant chacune un mélange gazeux NO/N₂, à savoir ici un mélange gazeux NO/N₂ contenant par exemple 800 ppmv de NO (reste N₂), alimentent en mélange NO/N₂, le dispositif de délivrance de NO 1, via des lignes d'amenée de gaz 30, tel que des tuyaux ou conduits flexibles ou analogues, qui peuvent être équipées de dispositifs de régulation et/ou de suivi de la pression de gaz 31, tels que détendeur de gaz, manomètres... Les bouteilles 21 sont équipées de robinet 4 de distribution du gaz.

Les lignes d'amenée de gaz 30 sont reliées à une ou plusieurs entrées de gaz 3 du dispositif de délivrance de NO 1 qui alimentent un passage de gaz interne servant à acheminer le gaz au sein du dispositif de délivrance de NO 1, c'est-à-dire dans le boitier 2 ou la carcasse externe du dispositif de délivrance de NO 1.

Le dispositif de délivrance de NO 1 comprend aussi une entrée d'oxygène 33 reliée fluidiquement, via une ligne d'amenée d'oxygène 34, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier.

Le ventilateur médical 23 qui fournit un flux de gaz respiratoire contenant une proportion élevée d'oxygène, tel de l'oxygène pur ou un mélange oxygène/azote (N₂/O₂), par exemple contenant au moins 60 %vol. d'O₂, et le dispositif de délivrance de NO 1 sont en communication fluidique avec la branche inspiratoire 22 du circuit patient 29, de manière à lui fournir le mélange gazeux à base de NO à acheminer jusqu'au patient, lequel mélange gazeux est formé par mélange du flux provenant du ventilateur médical 23 et du flux contenant le NO, i.e. le mélange gazeux NO/N₂, délivré par le dispositif de délivrance de NO 1. Le ventilateur 23 comprend des moyens de réglage, telles une ou des touches ou analogues, permettant de fixer notamment la FiO₂ souhaitée mais aussi à régler les caractéristiques du flux délivré, par exemple débit, pression et/ou autres.

Le dispositif de délivrance de NO délivre ou injecte le mélange NO/N₂, ici à 800 ppmv de NO, dans la branche inspiratoire 22, via un conduit ou ligne d'injection 37, de manière à injecter (en 37a) le flux de NO/N₂ dans le flux d'O₂ ou de mélange oxygène/azote délivré par le ventilateur médical 23 et véhiculé par la branche inspiratoire 22, aussi appelée ligne d'alimentation en gaz.

La branche inspiratoire 22 comprend en outre un humidificateur de gaz 24 agencé en aval du site 37a où se fait l'injection de NO dans la branche inspiratoire 22 du circuit patient 29. Il permet d'humidifier le flux de gaz, e.g. mélange NO/N₂/O₂, avant qu'il ne soit inhalé par le patient à traiter, au moyen d'une interface respiratoire, typiquement une sonde d'intubation trachéale 25.

La branche expiratoire 27 constitue une ligne de récupération des gaz expirés par le patient. Elle est raccordée à la branche inspiratoire 22 via une pièce de raccordement 28, de préférence une pièce en Y, en définissant ainsi le circuit patient 29.

La branche inspiratoire 22 est raccordée fluidiquement, en amont, à un port de sortie 23a du ventilateur médical 23 de manière à récupérer et acheminer le gaz, typiquement de l'O₂ ou un mélange N₂/O₂ délivré par le ventilateur médical 23, alors que la branche expiratoire 27 est raccordée fluidiquement, en aval, à un port d'entrée 23b du ventilateur médical 23 de manière à retourner au ventilateur médical 23, tout ou partie du débit des gaz expirés par le patient, c'est-à-dire les gaz riches en CO₂.

La branche expiratoire 27 peut comprendre un ou plusieurs composants optionnels, comme par exemple un dispositif d'élimination du CO₂ 26, i.e. un piège à CO₂, tel un bac à chaud ou autre, permettant d'éliminer le CO₂ présent dans les gaz expirés par le patient ou un filtre ou autre.

Optionnellement, un capteur de débit 36, par exemple du type à fil chaud ou à différentiel de pression, est agencé sur la ligne d'alimentation en gaz, c'est-à-dire la branche inspiratoire 22, entre le ventilateur 23 et l'humidificateur 24, et est relié au dispositif de délivrance de NO 1, via une ligne de mesure de débit 35. Cet agencement set à mesurer le débit de gaz délivré par le ventilateur 23, tel de l'O₂ ou un mélange N₂/O₂, et circulant dans la branche inspiratoire 22, en amont du site 37a de raccordement du conduit ou ligne d'injection 37 où se fait le mélange NO/N₂/O₂. Ceci permet de mieux réguler la délivrance du flux de NO par le dispositif de délivrance de NO 1.

Une ligne de prélèvement de gaz 38 relie fluidiquement la branche inspiratoire 22 au dispositif de délivrance de NO 1. Elle vient se raccorder fluidiquement (en 38a) à la branche inspiratoire 22, entre l'humidificateur 24 et la pièce de jonction 28, i.e. pièce en Y, typiquement à proximité immédiate de la pièce de jonction 28, et par ailleurs à un port d'entrée 102 du dispositif de délivrance de NO 1, par exemple un port porté par un connecteur, raccord ou analogue permettant le raccordement de la ligne de prélèvement de gaz 38, tel un tuyau flexible ou analogue. Elle permet de prélever des échantillons de gaz dont il convient de vérifier la conformité, et de les convoyer jusqu'au dispositif de délivrance de NO 1 où ils sont analysés dans un analyseur de gaz interne.

En particulier, il convient de vérifier que leur composition (i.e. posologie) est conforme à celle du mélange gazeux NO/O₂/N₂ souhaité devant être administré au patient, notamment pour s'assurer qu'il ne contient pas de quantité excessive d'espèces NO₂ toxiques, que sa teneur en oxygène n'est pas hypoxique et que sa teneur en NO correspond à la posologie souhaitée. Cette vérification de conformité se fait classiquement au moyen de moyens de mesure dédiés, typiquement des capteurs de NO₂, de NO et d'O₂, qui doivent eux-mêmes être calibrés périodiquement, par exemple toutes les semaines.

L'appareil de délivrance de NO 1 comprend, de manière classique, un boitier rigide, par exemple en polymère, traversé par un passage de gaz interne (non visible), tel un conduit de gaz ou analogue, pour acheminer le flux de NO/N₂ amené par la (ou les) ligne d'amenée de gaz 30 qui est alimentée par les bouteilles de mélange de NO/N₂ 21. Le passage de gaz interne relie fluidiquement l'entrée (ou les entrées) de gaz 3 du dispositif de délivrance de NO 1 à la ligne d'injection 37 de manière à convoyer le flux gazeux à base de NO entre eux.

De façon classique, des moyens à valve (non montré), i.e. un (ou des) dispositif à valve, par exemple une pluralité d'électrovannes agencées en parallèle ou une ou des (électro)vannes proportionnelles, sont agencés sur le passage de gaz interne pour contrôler le flux gazeux qui y circule en direction de la ligne d'injection 37. Les moyens à valve sont commandés par des moyens de pilotage, i.e. un (ou des) dispositif de pilotage, agencés dans le boitier, typiquement une carte électronique comprenant un (ou plusieurs) microprocesseur(s), typiquement un (ou des) microcontrôleur, mettant en oeuvre un ou des algorithmes.

Les moyens de pilotage permettent notamment d'ajuster ou contrôler le débit de gaz en pilotant les moyens à valve, typiquement d'ouvrir ou fermer cette ou ces vannes, pour obtenir un débit de gaz déterminé et/ou calculé par les moyens de pilotage à partir d'une valeur réglée/fixée par l'utilisateur, et en fonction du débit de gaz, i.e. air, délivré par le ventilateur 23 et mesuré par le capteur de débit 36 qui est relié au dispositif de délivrance de NO 1, par la ligne de mesure de débit 35. Les mesures de débit du flux délivré par le ventilateur 23 et circulant dans la branche inspiratoire 22 sont fournies aux moyens de pilotage.

Le passage de gaz interne peut aussi comprendre un (ou des) débitmètre (non représenté) agencé en amont et/ou en aval des moyens à valve, pour déterminer le débit de gaz à base de NO circulant dans le dispositif de délivrance de NO 1. Le débitmètre peut être du type à différentiel de pression, à fil chaud ou autre. Il coopère avec les moyens de pilotage pour leur fournir, là encore, des mesures de débit du flux de NO/N₂.

Par ailleurs, le dispositif de délivrance de NO 1 comprend aussi une interface graphique utilisateur ou IGU comprenant un afficheur graphique 7, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dose de NO...), ou toute autre information ou donnée utile au personnel soignant, notamment la dose de NO souhaitée dans le mélange final NO/O₂/N₂.

Les moyens de pilotage (non visibles) comprenant par exemple une carte de commande électronique et une unité de contrôle à microprocesseur, typiquement un microcontrôleur ou analogue. Ils permettent de contrôler ou commander tous les éléments électromécaniques de l'appareil de délivrance de NO 1. Plus précisément, la carte de commande intègre préférentiellement l'unité de contrôle et est configurée pour commander et par ailleurs analyser les signaux provenant des différents composants de l'appareil de délivrance de NO 1, tels que pompe, capteurs 32...

L'alimentation électrique de l'appareil de délivrance de NO 1, en particulier des composants nécessitant du courant électrique pour fonctionner, tels les moyens de pilotage, l'afficheur graphique 7..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant.

L'utilisateur peut par exemple sélectionner ou fixer, via l'IGU de l'appareil de délivrance de NO 1, la teneur en NO à administrer au patient, par exemple 20ppmv, et les moyens de pilotage pilotent alors l'appareil de délivrance de NO 1 pour délivrer le mélange NO/N₂ à un débit qui convient pour obtenir le mélange NO/N₂ et air (ou O₂/N₂) à la bonne concentration. Ceci se fait notamment grâce aux valeurs provenant du capteur de débit 32 qui mesure le débit (ou des pression) du flux d'air (ou O₂/N₂) provenant du ventilateur 23.

Typiquement, le mélange final fourni au patient qui contient du NO à la concentration souhaitée, de l'oxygène et de l'azote, contient en général moins de 40 ppmv de NO, par exemple de l'ordre de 10 à 20 ppmv environ.

D'une façon générale, la présente invention est particulièrement bien adaptée au traitement des patients SDRA sévères présentant des critères d'ECMO et ce, quelle que soit l'étiologie du SDRA, lesquels sont sous ventilation artificielle et intubés, i.e. sous ventilation invasive, typiquement des patients en réanimation ou analogue. Avantageusement, le iNO est administré rapidement après intubation du patient, de préférence dans les 24h après intubation.

## Revendications

1. Médicament gazeux contenant du monoxyde d'azote (NO) gazeux pour une utilisation dans le traitement d'un patient atteint d'un syndrome de détresse respiratoire aiguë (SDRA) comprenant une administration par inhalation dudit médicament gazeux audit patient, **caractérisé en ce que** le patient est un adulte atteint d'un SDRA sévère **se caractérisant par** :
i) un index de sévérité tel que : PaO₂/FiO₂ ≤ 80 mmHg
ou
ii) une pression partielle de dioxyde de carbone (PaCO₂) et un pH sanguin tels que : PaCO₂ ≥ 60 mm Hg et pH < 7.25,
où : PaO₂, PaCO₂ et/ou pH sont mesurés chez le patient avant administration du médicament gazeux et FiO₂ est la quantité d'oxygène fournie au patient.

2. Médicament pour une utilisation selon la revendication 1, **caractérisé en ce qu'**il est formé d'un mélange de NO et d'azote.

3. Médicament pour une utilisation selon l'une des revendications 1 ou 2, **caractérisé en ce qu'**il est formé d'un mélange de NO et d'azote contenant de 200 à 2000 ppmv de NO, de préférence entre de 200 à 1000 ppmv de NO.

4. Médicament pour une utilisation selon l'une des revendications 1 à 3, **caractérisé en ce qu'**il est formé d'un mélange de NO et d'azote contenant entre 400 et 900 ppmv de NO, le reste étant de l'azote.

5. Médicament pour une utilisation selon la revendication 1, **caractérisé en ce que** le patient est un adulte âgé d'au moins 18 ans, de préférence âgé d'au moins 40 ans.

6. Médicament pour une utilisation selon l'une des revendications 1, 3 ou 4, **caractérisé en ce qu'**il est formé d'un mélange de NO et d'azote contenant 800 ppmv de NO, le reste étant de l'azote.

7. Médicament pour une utilisation selon l'une des revendications 1 à 4 ou 6, **caractérisé en ce qu'**il est conditionné dans une bouteille de gaz à une pression d'au moins 150 bar abs ou est fourni par un dispositif générateur de NO.

8. Médicament pour une utilisation selon la revendication 1, **caractérisé en ce que** le patient est atteint d'un SDRA sévère **se caractérisant par** un index de sévérité tel que : 30 mHg ≤ PaO₂/FiO₂ ≤ 80 mmHg.

9. Médicament pour une utilisation selon la revendication 1, **caractérisé en ce que** le patient est atteint d'un SDRA sévère **se caractérisant par** un index de sévérité tel que : 40 mmHg ≤ PaO₂/FiO₂ ≤ 80 mmHg.

10. Médicament pour une utilisation selon la revendication 1, **caractérisé en ce que** le patient présente une ou des comorbidités choisies parmi une hypertension, un diabète, une immunodéficience et une bronchopneumopathie chronique obstructive (BPCO).

11. Médicament pour une utilisation selon la revendication 1, **caractérisé en ce qu'**il est administré par inhalation pendant plusieurs heures, de préférence entre 24h et 96h.

12. Médicament pour une utilisation selon la revendication 1, **caractérisé en ce qu'**il est administré par inhalation au moyen d'une sonde d'intubation trachéale

13. Médicament pour une utilisation selon la revendication 1, **caractérisé en ce qu'**il est dilué avec un gaz respiratoire contenant de l'oxygène, avant administration au patient.

14. Médicament pour une utilisation selon la revendication 1, **caractérisé en ce que** le patient est un adulte présentant, avant traitement par NO, une hypoxémie réfractaire.

15. Médicament pour une utilisation selon la revendication 1, **caractérisé en ce que** le patient est alité.
